# EUROPEAN PATENT SPECIFICATION

(11) **EP 3 968 899 B1**
(45) Date of publication and mention of the grant of the patent: **31.07.2024**
(21) Application number: 19726890.7
(22) Date of filing: 15.05.2019
(51) Int. Cl.: A61F 2/08, A61F 2/24

(54) **FELT MATERIAL FOR REPAIRING OR AUGMENTING HUMAN OR ANIMAL SOFT TISSUES**
FILZMATERIAL ZUR REPARATUR ODER VERGRÖSSERUNG VON MENSCHLICHEN ODER TIERISCHEN WEICHTEILEN
MATÉRIAU EN FEUTRE POUR RÉPARATION OU AUGMENTATION DE TISSUS MOUS HUMAINS OU ANIMAUX

(43) Date of publication of application: 23.03.2022
(73) Proprietor: ZuriMED Technologies AG, 8008 Zürich (CH)
(72) Inventor: BACHMANN, Elias, 8004 Zürich (CH); LI, Xiang, 8126 Zumikon (CH); SNEDEKER, Jess G., 8008 Zürich (CH); MEYER, Louisa, 8032 Zürich (CH); MEYER, Dominik C., 8032 Zürich (CH)
(74) Representative: Vossius & Partner Patentanwälte Rechtsanwälte mbB
(86) International application number: PCT/CH2019/000015
(87) International publication number: WO 2020/227838

(56) References cited:
- EP-A1- 2 404 557
- WO-A1-2009/005527
- WO-A1-2010/141906
- WO-A1-2016/044762
- US-A1- 2010 292 791
- US-A1- 2012 197 282
- US-B1- 7 252 832

## Description

### BACKGROUND OF THE INVENTION

### 1. Field of the Invention

The invention relates to a set of surgical instruments for attaching the felt material to soft tissue according to the preamble of claim 1.

Every year, more than 30 million musculoskeletal injuries are registered worldwide from which nearly half of them involve tendons and ligaments. Common tendon injuries are mostly related to the Achilles and rotator cuff tendons of the shoulder. In the last years more and more scaffold devices, such as extracellular matrix or synthetic grafts were used to mechanically and/or biologically augment repair sites to avoid suture pull-out or elongation which could result in failure. Up to now, sutures are indispensable for medical surgeries for guaranteeing tissue regeneration. The threads used for sutures can vary, depending on the location of the surgery and the targeted tissue. Not only mechanical properties of sutures and knotting techniques are essential for a good outcome. Especially early failure of repaired tendons and ligaments are caused by suture or knot failure where the suture pulling out of the tendon or through the bone. To ideally repair a soft-tissue, high initial fixation strength should be achieved to allow minimal gap formation and mechanical stability to allow a solid healing.

### 2. Description of the Related Art

A medical implant for repairing tendons and ligaments is known from US 2008/0188936 A1 [Ball et al.]. This known medical implant comprises an anchor structure attachable to bone by means of bone anchors and a tension member made of a patch material as for example a woven mesh, fabric made by weaving, knitting, braiding or felting fibers. During surgery one end of the tension member is attached to a tendon and then a tension force is applied to the other end of the tension member, which end is then engaged under tension with the anchor structure. A drawback of this known medical implant is that the implant is attached to soft tissue and/or bone at each of its ends only so that a mechanical connection of the implant and the soft tissue or bone is achieved in a small area only.

US 7,252,832 B1 discloses a felt for repairing soft tissue defects comprising a membranous collagen substrate and a bioresorbable fiber felted onto the collagen substrate. Methods of preparing a felt and methods of repairing soft tissue damage with a felt are also provided.

WO 2016/044762 A1 discloses apparatuses and methods for fabricating tubular structures from a combination of fibrous materials for use in, for example, tissue engineering scaffold applications. These materials may also be useful in other biological or non-biological applications in which such tubular fibrous structures may be applicable, examples including conventional medical devices, filters, fiber optics, cable wraps, geotextiles, batteries, fuel cells, armor, and other diverse applications.

WO 2009/005527 A1 discloses suture passers for suturing tissue in a continuous manner by passing a suture attached to a suture shuttle through. A suture passer may include a first jaw, a second jaw, and a tissue penetrator that is retractable and extendable from the first jaw. The tissue penetrator may have a suture shuttle engagement region, and the second jaw may include a shuttle dock. The suture shuttle may be transferred between the first and second jaws as the tissue penetrator is extended from the first jaw and engages the second jaw. In some variations of the tissue passer, one or both jaws are tissue penetrating. In some variations, the jaws open in parallel allowing large tissue regions to be positioned between the jaws. Methods of using these devices are also described, as are systems and kits including these devices.

### BRIEF SUMMARY OF THE INVENTION

It is an object of the invention to provide a strong and durable mechanical connection between a felt material and a soft tissue over an extended area of the contact interface between the felt material and the soft tissue to be treated therewith.

The invention solves the posed problem with a set of surgical instruments for attaching the felt material to soft tissue comprising the features of claim 1.

According to the invention a strong mechanical connection between soft tissue and the felt material is achievable in a broad range of the contacting surface resulting in optimal stress distribution.

Further advantageous embodiments of the invention can be commented as follows:
The fibers of the felted patch are pushed or pulled through the second surface into the soft tissue by means of a needle, pin or blade comprising barbs. The barbs catch the fibers of the felted patch or matting and push them through the second surface into the soft tissue. The barbs can be punched, carved or cut into the needle, pin or blade.

In a further embodiment the barbs are spaced at intervals along the needle, pin or blade.

In another embodiment the needle comprises a tip portion and the barbs alternatingly face forward and backward with respect to the tip portion. This configuration permits the advantage that the fibers of the felted patch can be pushed or pulled through the second surface into the soft tissue.

In another embodiment some fibers of the felted patch or matting are pushed or pulled to penetrate through the second surface in the complete area covered by the second surface. The felted patch or matting and the soft tissue can be interwoven in the complete area covered by the second surface so as to form a strong mechanical connection between the felted patch or matting and the soft tissue. The felted patch or matting can be highly loaded in an axial direction, i.e. in a direction parallel to the second surface but can be peeled of if the felted patch or matting is loaded perpendicular to the soft tissue. By this means the felted patch or matting can be repositioned or removed if necessary or beneficial.

In yet another embodiment the second surface of the felt patch or matting is a continuous area, preferably a continuous and flat area. By this means the felted patch or matting is configured to continuously extend over a large contacting area. Depending on its application the felt patch or matting can be configured for a flat connecting area or can be configured as a tubular construct provided with a longitudinal gap to apply the construct e.g. to blood vessels.

In a further embodiment the felt material additionally to the fibers of the felt material comprises fibers or strings of monofilament or multifilament nature and of higher tensile strength or having a larger diameter, wherein the ratio between the tensile force exertable on an additional fiber or string and on a fiber of the felt material is minimum 1.5, preferably minimum 2.0. Therewith the advantage can be achieved that by means of the additional fibers of higher tensile strength or having a larger diameter a higher tensile force can be applied to the felted patch or matting so as to significantly improve the stiffness and patch stability. Furthermore, the complete construct can be reinforced for example if sutures are stitched through the felted patch. An additional advantage is that non-linear stiffnesses can be achieved which are more similar to the natural behavior of tendons and ligaments.

In a further embodiment the additional fibers or strings are in the form of single fibers, a net, a fan or a weave.

In again a further embodiment the felted patch or matting additionally comprises at least a second layer, wherein the at least one second layer is differently configured or comprises a different material than the felted patch or matting. This configuration permits the advantage that a layer providing an anchoring function to the assembly can be added, or a second layer e.g. in the form of a braided structure (mesh or net) to reinforce or augment the felted patch can be added.

In another embodiment the felt material before attachment to soft tissue is pre-stretched in at least one direction along the first and second surfaces of the felted patch or matting. An advantage of this embodiment is that better mechanical properties as higher stiffness of the matting or felted patch can be achieved. Further, the initial fixation strength can be improved to allow minimal gap formation and mechanical stability permitting a solid healing of the soft tissue, e.g. tendon or ligament.

In another embodiment the felt material before attachment to soft tissue is additionally modified by means of a suture arranged with a diagonal stitching pattern. By this means better mechanical properties as higher stiffness of the matting or felted patch can be achieved.

In another embodiment the felted patch or matting comprises one or more augmented zones which each comprise either fibers or strings of monofilament or multifilament nature and of higher tensile strength or having a larger diameter, or a second layer or at least one suture arranged with a diagonal stitching pattern. This configuration permits the advantage that the attachment of the felted patch or matting may be effected in the regions which are not augmented, e.g. not directly on the reinforcement second layer or stitching pattern so as to not destroy the fibers or sutures in the augmented regions.

In a further embodiment the felted patch or matting additionally comprises - before attachment to soft tissue - one or more sutures which are incorporated in the felt material.

In again a further embodiment the one or more sutures - before attachment of the felted patch or matting to soft tissue - protrude from the felted patch or matting so as to form an anchor member. For a direct anchoring function of the felted patch to another member such as bone or cartilage, the patch-suture construct can be equipped with one or several anchor elements which are connected to the felted patch with sutures or other connecting elements.

In another embodiment - before attachment of the felted patch or matting to soft tissue - one end of a braided textile, preferably of a suture is unraveled and reorganized to a felted textile and affixed to the felted patch or matting to form a second layer.

In another embodiment the fibers of the felt material are aligned. This permits the advantage that higher unidirectional stiffness properties of the felt patch or matting can be achieved compared to a common felt material with randomly arranged fibers.

In another embodiment the fibers of the felt material comprise barbs arranged along the fibers, wherein the barbs are preferably integral with the fibers of the felt material. By this means an improved felting effect can be achieved. If bigger monofilament fibers or more likely sutures are used (> Ø 0.3mm) they can be modified by making cleaves (e.g. in a 30° angle) sidewise to the suture. Those created dual-angle barbs allow the suture to pass smoothly through soft tissue in one direction - but prevent it from slipping back through the soft tissue and make it more likely that they get interwoven and stuck with other fibers of their kind.

In yet another embodiment the felt material is covered or soaked with drugs or chemical agents like antibiotics, biocompatible glue or collagen.

In a further embodiment the felted patch or matting comprises an additional layer of a biocompatible self-adhesive film. This configuration allows a first mechanical connection or later permanent connection of the felted patch or matting to the soft tissue onto which it is attached.

In a further embodiment - during attachment of the felted patch or matting to soft tissue - the needle is guided at an angle different from 90° with respect to the second surface of the felted patch or matting. By means of guiding the needle so as to push or pull the fibers of the felted patch or matting through the second surface at an angle different from 90° towards the surface of the soft tissue a high initial fixation strength can be applied to allow minimal gap formation and mechanical stability permitting a solid healing of the soft tissue, e.g. tendon or ligament.

In a further embodiment - during attachment of the felted patch or matting to soft tissue - the needle is subsequently or alternatingly guided at angles larger and smaller than 90° with respect to the second surface of the felted patch or matting. The fibers of the felt material are crosswise stitched oblique to the surface of the soft tissue.

In a further embodiment more than one felted patch or matting is attached to soft tissue. Several felted patches can be used to connect two tendon ends (e.g. on top and on bottom).

In another embodiment more than one needle is used to displace some of the fibers of the felted patch or matting to penetrate through the second surface into the soft tissue at the same time and preferably from two or more directions.

In another embodiment one or more rotating needles are used to displace some of the fibers of the felted patch or matting to helically penetrate through the second surface into the soft tissue.

According to a further aspect of the invention a set of instruments for attaching a felt material to soft tissue is provided, wherein the set of instruments comprises:
- a needle with barbs and a tip portion;
- a stitching device comprising means to affix the needle and a tubular member surrounding the needle;
- a clamping device configured in the form of forceps and comprising:
   - a first clamping member and a second clamping member each having a proximal end and a distal end, wherein the distal ends form opposing first and second jaws suitable to abut soft tissue; wherein
   - the first jaw is frame-like configured permitting the tip portion of the needle to penetrate the first jaw in the range of the first surface of the felted patch or matting and holding means to hold the felted patch or matting; and wherein
   - the second jaw comprises a cavity open towards the first jaw to receive the tip portion of the needle.

In a further embodiment the stitching device comprises a drive mechanism, wherein the tubular member has a longitudinal axis and the drive mechanism is configured to displace the needle back and forth along the longitudinal axis of the tubular member.

In a further embodiment the first jaw comprises an opening, wherein the opening permits the tip portion of the needle to penetrate the first jaw in the range of the first surface of the felted patch or matting.

In another embodiment the second jaw comprises a mesh covering the cavity, wherein the mesh has openings permitting the needle to pass there through.

In another embodiment the second jaw comprises a plurality of brush fibers arranged in the cavity permitting the needle to pass between the brush fibers.

In again another embodiment the clamping device comprises a holder suitable to be attached to the stitching device, wherein the holder is configured to hold the felted patch or matting and to guide the needle at a selectable angle.

In a further embodiment the drive mechanism is attachable to an electric, pneumatic or hydraulic drive unit.

In a further embodiment the drive mechanism comprises means to adjust an amplitude of the back and forth movement of the needle. By this means the advantage can be achieved that dependent on the size and application different depths of needle penetration into the felted patch and soft tissue can be adjusted by the user. This is particularly useful in combination with a needle which has a defined transportation distance of the fibers that are pushed into the tissue particularly, if there is just a single series of hooks near the tip of the needle.

In a further embodiment the drive mechanism or a drive unit attached to the stitching device comprises means to rotate the needle about the longitudinal axis of the tubular member. By this means rotating or helically penetrating needles can be used with variable rotation of the needle during insertion or extraction.

In a further embodiment the tubular member comprises a hollow ball-shaped front end. This configuration permits the advantage that a better contact surface at the front end is achieved which particularly permits to prevent tilting or tipping over of the tubular member when contacting the surface of the felted patch or matting.

In another embodiment the barbs of the needle are rounded. By this means the cleaning, washing and sterilization processes of the needle are improved so as to provide needles which are particularly suitable for surgical use.

### A BRIEF DESCRIPTION OF THE DRAWINGS

Several embodiments of the invention will be described in the following by way of example and with reference to the accompanying drawings in which:
Fig. 1 illustrates a lateral view of an embodiment of a felt patch or matting made of the felt material according to the invention during its affixation to a tendon by means of a needle;
Figs. 2A-2G illustrate each a perspective view of a differently configured felt patch or matting made of the felt material according to invention;
Fig. 3 illustrates a perspective view of an embodiment of a set of surgical instruments for use with the felt material according to the invention in a method of repairing or augmenting human or animal soft tissue;
Figs. 4A, 4B illustrate lateral views of different embodiment of needles of the embodiment of a set of surgical instruments according to fig. 3;
Fig. 5 illustrates a sectional view of the jaws of the clamping device of the embodiment of a set of surgical instruments of fig. 3;
Fig. 6 illustrates a perspective view of another embodiment of a set of surgical instruments for use with the felt material according to the invention in a method of repairing or augmenting human or animal soft tissue;
Fig. 7 illustrates a schematic view of a first example for an application of an embodiment of a felt patch or matting made of the felt material according to the invention in the case of soft tissue repair/reconstruction; and
Fig. 8 illustrates a schematic view of a second example for an application of an embodiment of a felt patch or matting made of the felt material according to the invention in the case augmentation of a rotator cuff tendon.

### DETAILED DESCRIPTION OF THE INVENTION

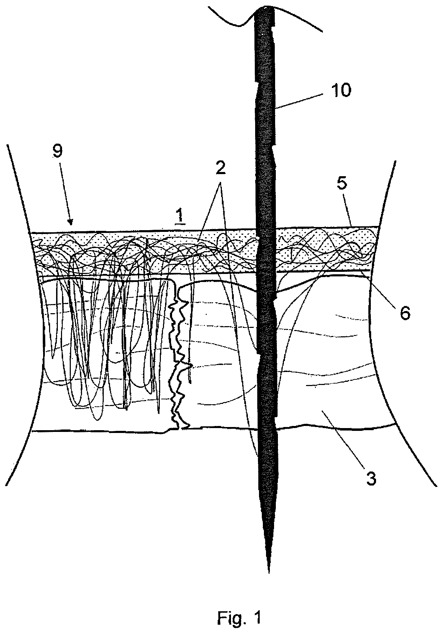

Fig. 1 illustrates an embodiment of the felt material 1 comprising a multitude of fibers 2 for use in a method of repairing or augmenting human or animal soft tissue 3 according to the invention. The felt material 1 is in the form of a matting or felted patch 9 which comprises a first surface 5 and oppositely arranged a second surface 6 for contacting a surface of soft tissue 3. In use the felted patch 9 or matting is affixed to soft tissue, e.g. a tendon or ligament by applying a method of repairing or augmenting human or animal soft tissue 3 comprising the steps of: A) positioning a felt material 1 in the form of a matting or felted patch 9 comprising a first surface 5 and oppositely arranged a second surface 6 for contacting a surface of soft tissue 3 onto a patient's soft tissue 3 and B) repeatedly advancing a needle 10 through the felted patch 9 or matting, so that some of the fibers 2 of the felted patch 9 or matting are pushed or pulled through the second surface 6 into the soft tissue 3 by means of the needle 10 to produce a firm connection between the felt material 1 and the soft tissue 3.

The above method using a felted patch 9 or matting can be used to reinforce the surrounding soft tissue as a preparation for the use of a conventional suture stitch (tendon-patch). Alternatively, it can also be used to fasten the felted patch 9 to connect it directly to a second target organ (for example tendon-patch-tendon or tendon-patch-bone). Another application is to fasten the felted patch 9, embodied with sutures and or an anchoring element for further fixation to a second structure such as soft tissue (tendon-patch & suture) or bone (tendon-patch & suture-bone anchor).

The felt material 1 applied according to the above method can be used for the repair, augmentation or fixation of anatomical structures such as: suture of collagenous tissues such as tendons or fascias, ligament reconstructions (collateral ligaments, cruciate ligaments, etc.), subcutaneous sutures, conventional suturing of skin closures, skeletal muscle, heart muscle and valves, hollow organs (large vessels, bladder, esophagus, possibly intestine).

Depending on its application:
- the felted patch 9 or matting for soft tissue fixation or augmentation as illustrated in fig. 1 may have different shapes, material components or even be a construct or compound of different materials or devices (e.g. patch interwoven with a mesh and sutures). The fibers 2 can either be from natural origin basis (natural fibers, cotton, silk etc.), synthetic [Polypropylene (PP), Polyamide (PA), Polytetrafluoroethylene (PTFE) Polyethylene (PE), Ultra-high-molecular-weight polyethylene (UHMWPE, UHMW), Polyethylenterephthalat (PET)], or other high strength fibers, etc.), metallic (Stainless steel wool, titanium wool, magnesium wool) or a combination of those. The fibers 2 can be used as a permanent implant or be biodegradable [poly-L-lactic acid (PLLA), poly-glycolic-acid (PGA), etc.)], combinations thereof and also be combined with non-degradable fibers 2. The felted patch 9 may in part or entirely also contain fibers 2 of biological origin such as collagen fibers or hair;

- the felted patch 9 or matting can have a thickness between 0.2- 20 mm, preferably 0.5-3 mm, and most preferably 1-2 mm. The felted fibers 2 can either be randomly aligned (like it is done in conventional felt production) or the fibers 2 can be aligned to achieve unidirectional stiffness properties of the felt material 1. For an improved felting effect, fibers 2 can be modified with barbs; and
- the fibers 2 of the felt material 1 can have a variable length of 2-500 mm and mixture of fibers 2 thereof with a thickness between 1-1000 µm, preferably 5-200 µm. Length, thickness, coating and fabrication of fibers 2 influence the internal structure of the felt material 1 and ultimately the augmentation effect. Changing these parameters and felting stiches and patterns during production of the felted patch 9 influence for example, how much force is necessary to peel of the felted patch 9 from the soft tissue 3 or influence variable directional stiffnesses of the felted patch 9. This enables a spectrum of degree of fineness of fibers 2 that can vary between 0.9-17 dtex with fiberlengths between 1-90 mm.

To get better mechanical properties in terms of higher stiffness of the construct, the felted patch 9 can be pre-stretched and or additionally modified with a diagonal stitching pattern. Furthermore, the felted patch 9 or matting can be covered or soaked with drugs or chemical agents such as antibiotics, a biocompatible glue, collagen or a layer of biocompatible self-adhesive film to allow a first mechanical connection or later permanent connection to the soft tissue 3 on which it is felted on.

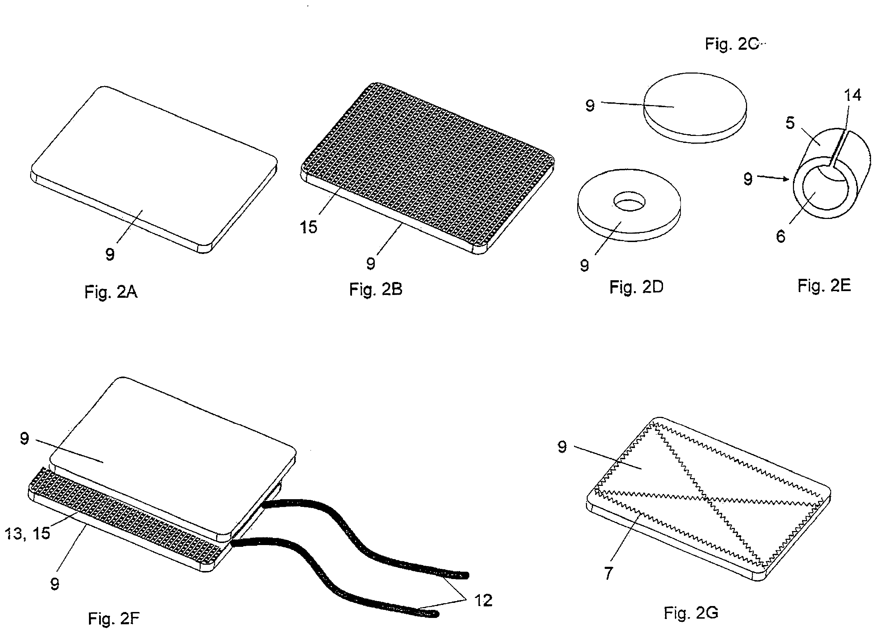

As illustrated in figs. 2A - 2G the felted patch 9 or matting can have - besides basic geometrical shapes of the felted patch 9 or matting such as round or rectangular patches (figs. 2A, 2C and 2D) - special configurations such as rings as illustrated in fig. 2D (e.g. for heart valve repair) or tubes including a longitudinal gap 14 as illustrated in fig. 2E (similar like a catheter) can be used for repair of hollow organs such as blood vessels. Alternatively, other geometrical shapes like concave and convex surfaces may also be used.

According to the alternative embodiments illustrated in figs. 2B and 2F the felted patch 9 comprises a second layer 15 so as to form an assembly of different layers which improves the felted patch 9 or matting and adds an anchoring function. Alternatively, to improve stiffness and patch stability stronger fibers 2 or strings of monofilament or multifilament nature in the form of single fibers, a net, fan or weave or is similar shapes can be incorporated into the felted patch 9 or matting. For this effect the ratio between the tensile force exertable on an additional fiber or string and on a fiber 2 of the felt material 1 is minimum 1.5, preferably minimum 2.0. This also allows to reinforce the construct for example if sutures are stitched through the felted patch 9 or matting. Similarly sutures (round or flat) can be incorporated into the felted patch 9 or matting. Alternatively, for a direct anchoring function of the felted patch 9 or matting to another member such as bones or cartilage, the patch-suture construct can be equipped with one or several anchor elements (not shown) which are connected to the felted patch 9 or matting with sutures or other connecting elements. Alternatively or additionally, as illustrated in fig. 2F one end of braided textiles (e.g. sutures 12) is unraveled, remodelled to a felted textile 13 and attached to the felted patch 9 or matting as a second layer 15.

Another alternative embodiment is illustrated in Fig. 2G where the felted patch 9 or matting comprises an additional stitching pattern e.g. zig-zag stitches with a yarn or suture 7 having a diameter of exemplarily 0.1 mm and a stitching distance of 2 mm so as to contribute to a higher construct stiffness (> 100 N/mm). Depending on the stitching/sewing pattern different stiffness properties can be achieved. The diagonal pattern adds stability if diagonal stress is applied which is favorable for e.g. rotator cuff surgery suture bridge augmentation. A diagonal stitching pattern makes the construct more robust against shear stresses which can occur e.g. when the shoulder is rotated.

The embodiments of figs. 2B, 2F and 2G are configured to augment the felted patch 9 or matting with respect to its stiffness either through one or more additional augmenting second layers 15 or by additional suturing with a suture 7 (e.g. X-pattern, or stitched outer edges). A long, almost endless thread, suture 7 or additional fiber that gives the felted patch 9 or matting a structure thus results in an overall construct with better mechanical properties and prevents the individual fibers 2 of the felt material 1 from being pulled apart. The combination of individual filaments that can be felted with a felting needle 10 but which are weak on the construct and additional long, systematically sutured threads or sutures 7 make the felted patch 9 a capable implant. In various embodiments the felted patch 9 or matting can comprise zones which are not augmented, and other zones which are augmented (e.g., by an additional second layer 15 or suturing). The augmented zones are not penetrated by the felting needle 10 while the non-augmented zones are used to attach the felted patch 9 or matting to the soft tissue 3.

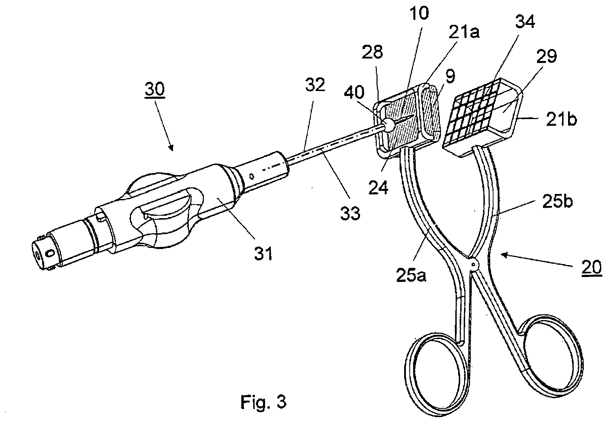

Figs. 3 - 5 illustrate an embodiment of a set of instruments according to the invention for attaching the felt material 1 to soft tissue 3. The set of instruments comprises:
- a needle 10 with a tip portion 16;
- a stitching device 30 comprising means to affix the needle 10, a tubular member 32 surrounding the needle 10 and a drive mechanism 31; and
- a clamping device 20 configured in the form of forceps and comprising a first clamping member 25a and a second clamping member 25b each having a proximal end and a distal end, wherein the distal ends form opposing first and second jaws 21a;21b suitable to abut soft tissue 3.

The first jaw 21a is frame-like configured permitting the tip portion of the needle 10 to penetrate the first jaw 21a in the range of the first surface 5 of the felted patch 9 or matting and holding means 28 to hold the felted patch 9 or matting. The second jaw 21b comprises a cavity 29 open towards the first jaw 21a to receive the tip portion of the needle 10. The tubular member 32 has a longitudinal axis 33 and the drive mechanism 31 is configured to displace the needle 10 back and forth along the longitudinal axis 33 of the tubular member 32. The frame-like first jaw 21a comprises an opening 24 which permits the tip portion of the needle 10 to penetrate the first jaw 21a in the range of the first surface 5 of the felted patch 9 or matting.

Furthermore, the tubular member 32 has a hollow ball-shaped front end 40 (e.g. with a diameter between 2 and 10 mm). By this means a better contact surface can be achieved and tilting or tipping over of the tubular member 32 when abutting the felted patch 9 can be prevented.

In a variety of embodiments the drive mechanism 31 of the stitching device 30 can either be induced manually, motorized or by means of pneumatic, magnetic or hydraulic aids. Alternatively, the stitching device 30 can be plugged on a motorized source. The stitching device 30 is configured as a single needle device but - alternatively can be consistent of many needles.

In a further embodiment the drive mechanism 31 or a drive unit (not shown) attached to the stitching device 30 comprises means to rotate the needle 10 about the longitudinal axis 33 of the tubular member 32. Therewith rotating or helically penetrating needles can be used with variable rotation of the needle 10 during insertion or extraction.

In another embodiment the drive mechanism 31 comprises means (not shown) to adjust an amplitude of the back and forth movement of the needle 10 so that dependent on the size and application different depths of needle penetration into the felted patch 9 or matting and soft tissue 3 can be adjusted by the user. This is particularly useful in combination with a needle 10 which has a defined transportation distance of the fibers 2 that are pushed into the soft tissue 3 particularly, if there is just single series of hooks near the tip of the needle 10. In another embodiment, a pair or series of needles can stich consecutively if several needles are used. Parallel stich patters or crosswise patterns can be achieved.

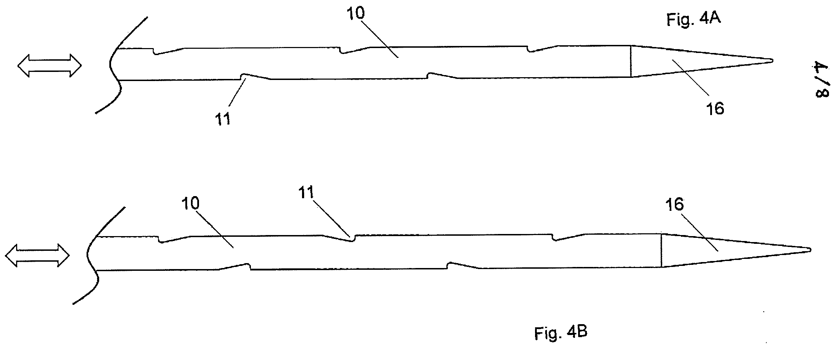

The shape of the needle 10 and the orientation of the felting barbs 11 can be configured to push the fibers 2 of the felt material 1 through the second surface 6 of the felted patch 9 or matting (fig. 4A) or to pull the fibers 2 of the felt material 1 through the second surface 6 of the felted patch or matting (fig. 4B).

Additionally, flat or round shaped needles 10 can be used dependent on preventing tissue damage. The felting barbs 11 can either be oriented in one direction or both linear (circular) directions. This will allow that fibers 2 of the felt material 1 are pushed and/or pulled in both directions (e.g. in a configuration were felted patches 9 are used on top and bottom of soft tissue 3). The felting barbs 11 can either be oriented side to side, in a gradual or circumferential pattern.

The stitching technique can be perpendicular to the soft tissue 3, so that the needle guidance is parallel. In another embodiment the needle guidance is in a certain angle towards the surface of the soft tissue. In another embodiment a crosswise stitching, oblique to the surface is performed. In an embodiment were several felted patches 9 are used to connect two tendon ends (e.g. on top and on bottom) several needles 10 can stich at the same time from two or several directions, each needle 10 equipped with barbs 11.

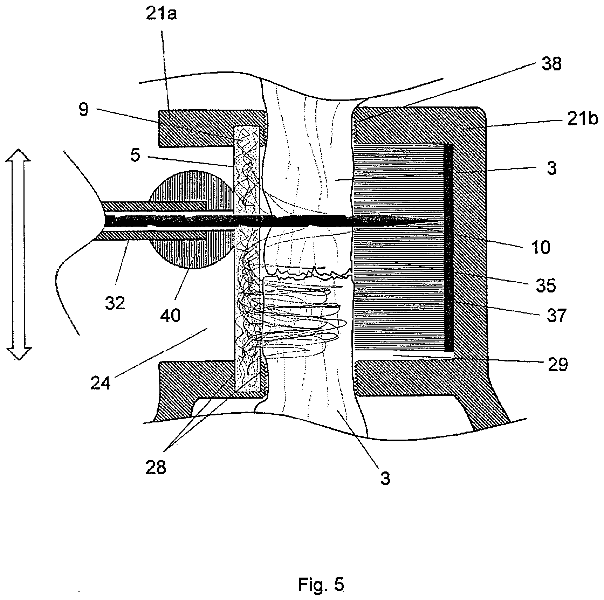

As illustrated in figs.3 and 5 the first jaw 21a comprises an opening 24, wherein the opening 24 permits the tip portion of the needle 10 to penetrate the first jaw 21a in the range of the first surface 5 of the felted patch 9 or matting. Furthermore, the second jaw 21b comprises a mesh 34 covering the cavity 29 (fig. 3), wherein the mesh 34 has openings permitting the needle 10 to pass there through. Alternatively, the second jaw 21b can comprise a plurality of brush fibers 35 (fig. 5) attached to a brush base 37 arranged in the cavity 29 instead of the mesh 34 so as to permit the needle 10 to pass between the brush fibers 35. The surface of the second jaw 21b which is opposite the first jaw 21a is provided with a teeth structure 38 to achieve a better grip of the soft tissue 3 (fig. 5). Alternatively, both opposing surfaces of the first and second jaws 21a, 21b can be provided with a teeth structure 38.

As illustrated in figs. 4A, 4B the needle 10 comprises barbs 11 which are oriented in a direction so that they catch and push or pull fibers 2 in the stitching direction of the needle 10. The soft tissue 3, in this example two torn ends of a tendon 4, are held in place together by a surgical or a specifically for the purpose devised clamp 20. The surgical clamp 20 has two jaws 21a; 21b with each a tip, while the tip of the first jaw 21a holds the felted patch 9 [flexible member] in place. The second jaw 21b of the surgical clamp 20 is spanned with a net or one or more brushes 22 which are pressed and therefore wrapped around the tendon 4. The brush structure allows the needle 10 to go through the brush hairs, while keeping the tendon 4 in position. Instead of a net, fine wires can be used which are aligned in parallel with a certain distance. The purpose of this brush 22, net or wire structure is to constrain the soft tissue 3 while allowing the needle 10 to stich through the soft tissue 3. The second jaw 21b of the surgical clamp 20 has a space 23 to allow the needle 10 to stich deep and protects surrounding soft tissue 3 from needle penetration. When the needle 10 penetrates the felted patch 9 [a flexible member] which comprises [made out] a multitude of loose fibers 2, the needle 10 pushes or pulls a number of these [those] fibers 2 into the [another] soft tissue 3 e.g. tendon 4. The deeper the needle 10 can stich through the soft tissue 3, the higher the chance, that many fibers 2 were pulled (also from more distant barbs 11 of the needle 10) through the soft tissue 3. If this process is repeatedly performed (2 times per second, preferably 5 times per second) the felted patch 9 and the soft tissue 3 get interwoven and a strong mechanical connection is achieved.

The needle 10 is, exemplarily but not limiting, provided with specially rounded barbs 11 which allows for improved reprocessing after the mechanical production of these needles 10 (cleaning, washing, sterilizing). This configuration is thus particularly suitable for medical applications.

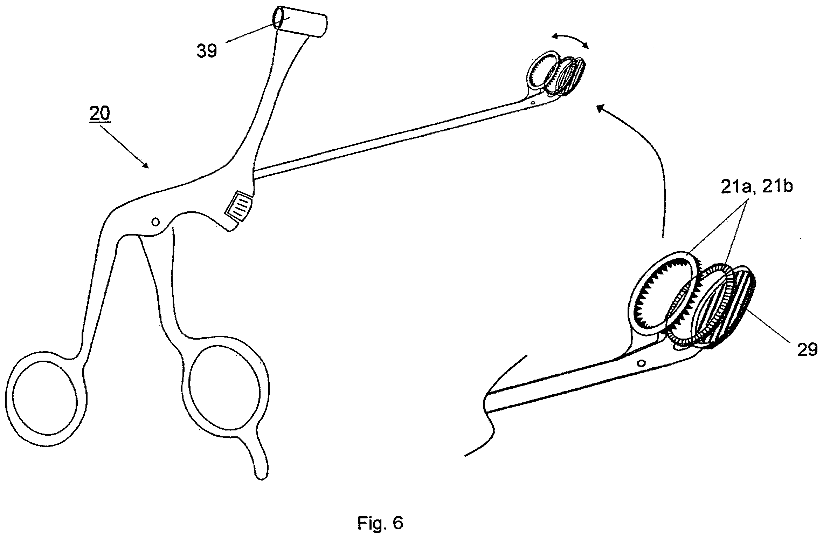

An alternative embodiment of the clamping device 20 for an arthroscopic indication is illustrated in fig. 6. The clamping device 20 comprises a positioning device 39 for the stitching device 30 (e.g. a guide tube for easier positioning of the stitching device 30). Furthermore, the first and second jaws 21a, 21b of the clamping device 20 are small enough to be transported through a surgical body opening with a diameter of 20mm. Alternatively, the surgical body opening may have a diameter of 10 mm or 6 mm.

During attachment of the felted patch 9 or matting, exemplarily but not limiting, more than 2000 fibers / cm² are pushed through the soft tissue 3. Stress which is applied on the felt construct will be equally distributed on those 2000 fibers which is very attractive for a better healing and against failure of sutures getting pulled tough. In terms of stress distribution of sutures/fibers the felting method according to the invention has an almost 1000-times better stress distribution than conventional suturing methods. Differently oriented felting fibers 2 (isotropic or orthotropic) could be beneficial for cell ingrowth, mimicking the underlying soft tissue 3. A layer of parallel, longitudinal fibers 2 promotes ingrowth of tendon cells while an orthotropic structure promotes ingrowth of heart muscle cells.

### Example 1 (soft tissue repair/reconstruction):

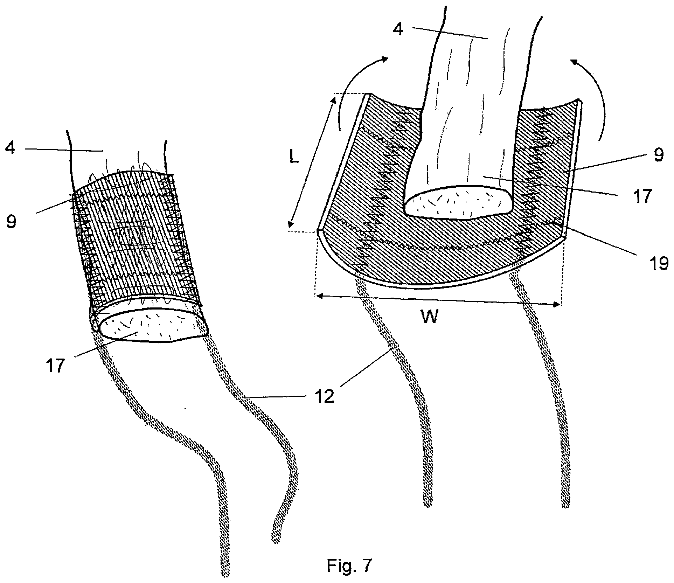

In this example illustrated in fig. 7, a tendon end 17 is attached to a suture 12 as a preparation step before surgical attachment of this graft into the body (e.g. ACL reconstruction Semitendinosus-tendon graft preparation, or biceps tenodesis):
A felted patch 9 with the dimension L x W (e.g. 40 x 40 mm) and a thickness of e.g. 3 mm is tightly enlaced/wrapped around a tendon end 17 and clamped together at its ends. Polyethyleneterephthalate (PET) fibers 2 of the felting patch 9 are a mix of different fiber length (20-60mm) with a thickness of 5 µm. The patch/suture construct comprises a tear strength of 250N when fully hydrated. The felt patch 9 has a reinforced stitching pattern with PET-yarn 19 (Ø=0.1mm) and sewed in sutures 12 (Fig. 7).

After that the yet loose tendon-patch construct is placed on a brush mattress and felted with a stitching device which allows to stich 2500 times per minute. The felted patch 9 is now felted onto the tendon 4 by moving and angle the stitching device over the top surface of the felted patch 9 for 30 seconds. After that, the tendon 4 is turned by 180° and the process is repeated with the bottom surface of the felted patch 9. This results in a 380° penetration of the needle 10 and a transportation of more than 10.000 fibers 2 into the tendon 4.

The felted tendon-patch-suture construct can now be implanted into e.g. a bone tunnel and sutures can be enlaced and knotted to a cortical fixation device or suture anchor. The felting method took 60 seconds and avoids time consuming suture (e.g. Mason-Allan stitching) of tendon ends 17, while minimizes the risk of suture pull out of the tendon.

### Example 2 (augmentation of a rotator cuff tendon):

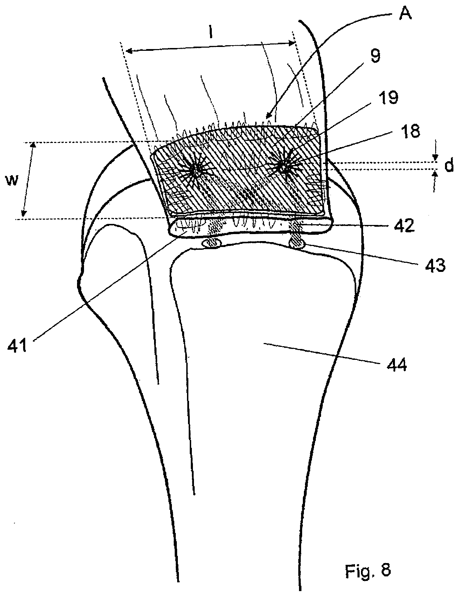

In example 2 as illustrated in fig. 8 a felted patch 9 with the dimensions w x l (20mm wide and 25mm long) and a thickness of 2mm is used. The felted patch 9 consists of Polyethyleneterephthalate (PET) fibers 2 of different fiber length (circa 40mm) with a thickness of 10 µm.

The felted patch 9 has two punched holes 18 with a diameter d of 1mm, which are reinforced directly at the outer rim of the punched hole 18 with a PET-yarn 19 (0=0.1mm) in a zigzag pattern, circular and concentric to the punched holes 18. This reinforces the felted patch 9 (if just a felt, comprising of a dense assembly of loose fibers is used, it cannot withstand higher forces [circa 50N]). By reinforcing with a stitching pattern with a yarn 19, the felted patch 9 gains higher stiffness (circa 200N/mm when fully hydrated). In a next step the felted patch 9 is placed on top of a rotator cuff tendon 41 and fixed by means of a clamp into the rotator cuff tendon 41. A 3-sided felting needle (cross-section looks like a triangle) each side having a length of 0.5mm is used. The total penetration depth of the needle is 20mm. The needle has a total of 6 barbs, each with a distance of 1mm separated and a barb depth of 0.1mm. A stitching device is used which allows to stich 2500 times per minute. The felted patch 9 is now felted onto the rotator cuff tendon 41 by moving the stitching device in a circular way around the punched holes 18, while avoiding to run over the reinforced zigzag pattern (else the barbs of the needle could destroy the yarn). This is done for 30 seconds, resulting approximately in a total of 1250 stitches. Assumed that more than one fiber 2 is pushed down into the soft tissue 3 by means of the needle a manifold of 1250 fibers are pushed town into the soft tissue (5x 1250 => 6000 fibers). In a last step two USP 2 suture 42 with suture needles and including suture anchors 43 (e.g. to be anchored in a humeral head 44) are used by penetrating the suture needle trough each of the punched holes 18.

If tension is now applied onto the sutures 42, cutting into the soft tissue is now reduced as the reinforced felted patch 9 distributes and absorbs punctual loading.

Although the invention has been described in conjunction with specific embodiments thereof, it is evident that many alternatives, modifications and variations will be apparent to those skilled in the art. Accordingly, it is intended to embrace all such alternatives, modifications and variations that fall within the scope of the appended claims.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately *or in* any suitable subcombination or as suitable in any other described embodiment of the invention. Certain features described in the context of various embodiments are not to be considered essential features of those embodiments, unless the embodiment is inoperative without those elements.

## Claims

1. Set of instruments for attaching a felt material (1) to a human or animal soft tissue (3), wherein the set of instruments comprises:
- a needle (10) with barbs (11) and a tip portion (16); wherein the barbs are configured to catch fibers of the felt material and to push or pull them through a surface of the felt material into the human or animal soft tissue;
- a stitching device (30) comprising means to affix the needle (10) and a tubular member (32) surrounding the needle (10); and
- a clamping device (20) configured in the form of forceps and comprising:
a first clamping member (25a) and a second clamping member (25b) each having a proximal end and a distal end, wherein the distal ends form opposing first and second jaws (21a;21b) suitable to abut soft tissue (3);
wherein the first jaw (21a) is frame-like configured permitting the tip portion of the needle (10) to penetrate the first jaw (21a) in the range of a first surface (5) of the felt material having a form of a felted patch (9) and holding means (28) to hold the felted patch (9); and
wherein the second jaw (21b) comprises a cavity (29) open towards the first jaw (21a) to receive the tip portion of the needle (10).

2. Set according to claim 1, wherein the stitching device (30) comprises a drive mechanism (31), wherein the tubular member has a longitudinal axis (33), and the drive mechanism (31) is configured to displace the needle (10) back and forth along the longitudinal axis (33) of the tubular member (32).

3. Set according to claims 2, wherein the drive mechanism (31) comprises means to adjust an amplitude of the back and forth movement of the needle (10).

4. Set according to claim 1, wherein the stitching device (30) comprises a drive mechanism (31), wherein the drive mechanism (31) is attachable to an electric, pneumatic or hydraulic drive unit (37).

5. Set according to one of claims 2 to 4, wherein the needle (10) comprises a tip portion (16) and the barbs (11) alternatingly face forward and backward with respect to the tip portion (16).

6. Set according to one of the previous claims, wherein the barbs are spaced at intervals along the needle.

7. Set according to one of the previous claims, wherein the clamping device (20) comprises a holder (36) suitable to be attached to the stitching device (30), wherein the holder (36) is configured to hold the felted patch (9) and to guide the needle (10) at a selectable angle.

8. Set according to one of the previous claims, additionally comprising: the felt material (1), the felt material comprising a multitude of fibers (2) for use in a method of repairing or augmenting human or animal soft tissues (3), wherein the felt material (1) is in the form of or felted patch (9) comprising a first surface (5) and oppositely arranged a second surface (6) for contacting a surface of soft tissue (3) the fibers (2) of the felted patch (9) being configured to be pushed or pulled through the second surface (6) into the soft tissue (3) producing a connection between the felt material (1) and the soft tissue (3).

9. Set according to claim 8, wherein the felt material additionally comprises one or more sutures (7) which are incorporated in the felt material (1).

10. Set according to claim 8 or 9, wherein the felt material (1) is covered or soaked with drugs or chemical agents, in particular antibiotics, biocompatible glue or collagen.

11. Set according to one of claims 8 to 10, wherein the second surface (6) of the felt material is a continuous area, preferably a continuous and flat area.

12. Set according to one of claims 8 to 11, wherein the felt material (1) additionally to the fibers (2) of the felt material (1) comprises monofilament or multifilament fibers or strings of higher tensile strength or having a larger diameter, wherein the ratio between the tensile force exertable on an additional fiber or string and on a fiber (2) of the felt material (1) is minimum 1.5, preferably minimum 2.0.

13. Set according to claim 12, wherein the additional fibers or strings are in the form of single fibers, a net, a fan or a weave.

14. Set according to one of claims 8 to 13, wherein the felted patch (9) additionally comprises at least one second layer (15), wherein the at least one second layer (15) is differently configured or comprises a different material than the felted patch (9).

## Patentansprüche

1. Instrumentenset zum Befestigen eines Filzmaterials (1) an einem menschlichen oder tierischen Weichgewebe (3), wobei das Instrumentenset aufweist:
- eine Nadel (10) mit Widerhaken (11) und einem Spitzenabschnitt (16); wobei die Widerhaken konfiguriert sind, Fasern des Filzmaterials zu ergreifen und sie durch eine Oberfläche des Filzmaterials in das menschliche oder tierische Weichgewebe zu drücken oder zu ziehen;
- eine Stichvorrichtung (30), die eine Einrichtung zum Befestigen der Nadel (10) und ein röhrenförmiges Element (32) aufweist, das die Nadel (10) umgibt; und
- eine Klemmvorrichtung (20), die in Form einer Zange ausgebildet ist und aufweist:
ein erstes Klemmelement (25a) und ein zweites Klemmelement (25b), die jeweils ein proximales Ende und ein distales Ende aufweisen, wobei die distalen Enden gegenüberliegende erste und zweite Backen (21a; 21b) bilden, die geeignet sind, an Weichgewebe (3) anzuliegen;
wobei die erste Klemmbacke (21a) rahmenförmig konfiguriert ist, was es ermöglicht, dass der Spitzenabschnitt der Nadel (10) die erste Klemmbacke (21a) im Bereich einer ersten Oberfläche (5) des Filzmaterials, das die Form eines Filzpatches (9) hat, und eine Halteeinrichtung (28) zu durchdringen, wobei die Halteeinrichtung das Filzpatch (9) hält; und
wobei die zweite Backe (21b) einen zur ersten Backe (21a) hin offenen Hohlraum (29) aufweist, um den Spitzenabschnitt der Nadel (10) aufzunehmen.

2. Set nach Anspruch 1, wobei die Stichvorrichtung (30) einen Antriebsmechanismus (31) aufweist, wobei das röhrenförmige Element eine Längsachse (33) aufweist und der Antriebsmechanismus (31) konfiguriert ist, die Nadel (10) entlang der Längsachse (33) des röhrenförmigen Elements (32) hin und her zu verschieben.

3. Set nach Anspruch 2, wobei der Antriebsmechanismus (31) eine Einrichtung aufweist, um eine Amplitude der Hin- und Herbewegung der Nadel (10) einzustellen.

4. Set nach Anspruch 1, wobei die Stichvorrichtung (30) einen Antriebsmechanismus (31) aufweist, wobei der Antriebsmechanismus (31) an eine elektrische, pneumatische oder hydraulische Antriebseinheit (37) anschließbar ist.

5. Set nach einem der Ansprüche 2 bis 4, wobei die Nadel (10) einen Spitzenabschnitt (16) aufweist und die Widerhaken (11) in Bezug auf den Spitzenabschnitt (16) abwechselnd nach vorne und nach hinten gerichtet sind.

6. Set nach einem der vorhergehenden Ansprüche, wobei die Widerhaken in Abständen entlang der Nadel angeordnet sind.

7. Set nach einem der vorhergehenden Ansprüche, wobei die Klemmvorrichtung (20) einen Halter (36) aufweist, der geeignet ist, an der Stichvorrichtung (30) angebracht zu werden, wobei der Halter (36) konfiguriert ist, den Filzpatch (9) zu halten und die Nadel (10) in einem wählbaren Winkel zu führen.

8. Set nach einem der vorhergehenden Ansprüche, das zusätzlich aufweist: das Filzmaterial (1), wobei das Filzmaterial mehrere Fasern (2) aufweist, zur Verwendung in einem Verfahren zum Reparieren oder Vergrößern von menschlichem oder tierischem Weichgewebe (3), wobei das Filzmaterial (1) die Form eines Filzpatches (9) aufweist, der eine erste Oberfläche (5) und eine gegenüberliegend angeordnete zweite Oberfläche (6) zum Berühren einer Oberfläche von Weichgewebe (3) aufweist, wobei die Fasern (2) des Filzpatches (9) konfiguriert sind, durch die zweite Oberfläche (6) in das Weichgewebe (3) geschoben oder gezogen zu werden, wodurch eine Verbindung zwischen dem Filzmaterial (1) und dem Weichgewebe (3) hergestellt wird.

9. Set nach Anspruch 8, wobei das Filzmaterial zusätzlich eine oder mehrere Nähte (7) aufweist, die in das Filzmaterial (1) eingearbeitet sind.

10. Set nach Anspruch 8 oder 9, wobei das Filzmaterial (1) mit Medikamenten oder chemischen Mitteln, insbesondere Antibiotika, biokompatiblem Klebstoff oder Kollagen, bedeckt oder getränkt ist.

11. Set nach einem der Ansprüche 8 bis 10, wobei die zweite Oberfläche (6) des Filzmaterials ein zusammenhängender Bereich, vorzugsweise ein zusammenhängender und ebener Bereich, ist.

12. Set nach einem der Ansprüche 8 bis 11, wobei das Filzmaterial (1) zusätzlich zu den Fasern (2) des Filzmaterials (1) Monofilament- oder Multifilament-Fasern oder Stränge mit höherer Zugfestigkeit oder größerem Durchmesser aufweist, wobei das Verhältnis zwischen der auf eine zusätzliche Faser oder Strang und auf eine Faser (2) des Filzmaterials (1) ausübbaren Zugkraft mindestens 1,5, vorzugsweise mindestens 2,0 beträgt.

13. Set nach Anspruch 12, wobei die zusätzlichen Fasern oder Stränge in Form von Einzelfasern, eines Netzes, eines Fächers oder eines Gewebes vorliegen.

14. Set nach einem der Ansprüche 8 bis 13, wobei der Filzpatch (9) zusätzlich mindestens eine zweite Lage (15) aufweist, wobei die mindestens eine zweite Lage (15) anders gestaltet ist oder ein anderes Material aufweist als der Filzpatch (9).

## Revendications

1. Ensemble d'instruments permettant de fixer un matériau de feutre (1) à un tissu mou humain ou animal (3), ledit ensemble d'instruments comprenant :
- une aiguille (10) avec des barbelures (11) et une partie de pointe (16) ; où les barbelures sont prévues pour accrocher des fibres du matériau de feutre et pousser ou tirer celles-ci à travers une surface du matériau de feutre dans le tissu mou de l'homme ou de l'animal ;
- un dispositif de piqûre (30) comprenant un moyen de fixation d'aiguille (10) et un élément tubulaire (32) entourant l'aiguille (10) ; et
- un dispositif de serrage (20) prévu sous la forme d'une pince et comprenant :
un premier élément de serrage (25a) et un deuxième élément de serrage (25b) ayant chacun une extrémité proximale et une extrémité distale, où les extrémités distales forment une première et une deuxième mâchoires opposées (21a; 21b) aptes à contacter les tissus mous (3) ;
où la première mâchoire (21a) est prévue en forme de cadre permettant à la pointe de l'aiguille (10) de pénétrer dans la première mâchoire (21a) au niveau d'une première surface (5) du matériau de feutre ayant la forme d'une pièce de feutre (9) et d'un moyen de maintien (28) pour maintenir la pièce de feutre (9) ; et
où la deuxième mâchoire (21b) comprend une cavité (29) ouverte vers la première mâchoire (21a) pour recevoir la partie de pointe de l'aiguille (10).

2. Ensemble selon la revendication 1, où le dispositif de piqûre (30) comprend un mécanisme d'entraînement (31), l'élément tubulaire présente un axe longitudinal (33), et le mécanisme d'entraînement (31) est prévu pour déplacer l'aiguille (10) d'avant en arrière le long de l'axe longitudinal (33) de l'élément tubulaire (32).

3. Ensemble selon la revendication 2, où le mécanisme d'entraînement (31) comprend des moyens de réglage de l'amplitude du mouvement avant-arrière de l'aiguille (10).

4. Ensemble selon la revendication 1, où le dispositif de piqûre (30) comprend un mécanisme d'entraînement (31), ledit mécanisme d'entraînement (31) pouvant être fixé à une unité d'entraînement électrique, pneumatique ou hydraulique (37).

5. Ensemble selon l'une des revendications 2 à 4, où l'aiguille (10) comprend une partie de pointe (16) et les barbelures (11) sont orientées de manière alternée vers l'avant et vers l'arrière par rapport à la partie de pointe (16).

6. Ensemble selon l'une des revendications précédentes, où les barbelures sont espacées le long de l'aiguille.

7. Ensemble selon l'une des revendications précédentes, où le dispositif de serrage (20) comprend un support (36) apte à être fixé au dispositif de piqûre (30), ledit support (36) étant prévu pour maintenir la pièce de feutre (9) et pour guider l'aiguille (10) suivant un angle sélectionnable.

8. Ensemble selon l'une des revendications précédentes, comprenant en outre : le matériau de feutre (1), ledit matériau de feutre comprenant une pluralité de fibres (2) destinées à être utilisées dans un procédé de réparation ou d'augmentation de tissus mous humains ou animaux (3), ledit matériau de feutre (1) se présentant sous la forme d'une pièce de feutre (9) comprenant une première surface (5) et une deuxième surface (6) opposée pour contacter une surface de tissu mou (3), les fibres (2) de la pièce de feutre (9) étant prévues pour être poussées ou tirées à travers la deuxième surface (6) dans le tissu mou (3), en réalisant une liaison entre le matériau de feutre (1) et le tissu mou (3).

9. Ensemble selon la revendication 8, où le matériau de feutre comprend en outre une ou plusieurs sutures (7) incorporées dans le matériau de feutre (1).

10. Ensemble selon la revendication 8 ou la revendication 9, où le matériau de feutre (1) est recouvert ou imbibé de médicaments ou d'agents chimiques, en particulier d'antibiotiques, de colle ou de collagène biocompatibles.

11. Ensemble selon l'une des revendications 8 à 10, où la deuxième surface (6) du matériau de feutre est une zone continue, de préférence une zone continue et plane.

12. Ensemble selon l'une des revendications 8 à 11, où le matériau de feutre (i) comprend, en plus des fibres (2) dudit matériau de feutre (1), des fibres ou des cordons monofilamentaires ou multifilamentaires ayant une résistance à la traction élevée ou de diamètre supérieur, le rapport entre la force de traction pouvant être exercée sur une fibre ou un cordon additionnels et sur une fibre (2) du matériau de feutre (1) étant d'au moins 1,5, de préférence d'au moins 2,0.

13. Ensemble selon la revendication 12, où les fibres ou cordons additionnels se présentent sous la forme de fibres simples, d'un réseau, d'un éventail ou d'une armure.

14. Ensemble selon l'une des revendications 8 à 13, où la pièce de feutre (9) comprend en outre au moins une deuxième couche (15), ladite au moins une deuxième couche (15) étant prévue différente, ou comprenant un matériau différent par rapport à la pièce de feutre (9).
